# EUROPEAN PATENT APPLICATION

(11) **EP 2 218 707 A1**
(43) Date of publication of application: **18.08.2010**
(21) Application number: 09152936.2
(22) Date of filing: 16.02.2009
(51) Int. Cl.: C07C 67/56, C07C 69/16

(54) **Process for the preparation of non-genotoxic Diacetylrhein (Diacerein)**

(71) Applicant: Evultis S.A., 6901 Lugano (CH)
(72) Inventor: Mazzola, Alessandro, 20149, MILANO (IT); Moiana, Silvia, 22063, CANTU-COMO (IT)
(74) Representative: Ahner, Francis

(57) **Abstract**

The present invention is directed to a process for producing non-genotoxic Diacetylrhein (Diacerein), comprising: i) transformation of raw Diacerein (or raw Rhein), into a water-soluble salt; ii) adsorption of the salt Diacerein (or Rhein) solution on a hydrophobic resin; iii) washing with an appropriate solvent to elminate the impurities (in particular the genotoxic impurities); iv) elution to recover Diacerein or Rhein; v) if the process is applied to Rhein, its transformation to Diacerein by acetylation; vi) acidification of purified Diacerein and its recovery, and drying.

The invention is also directed to non-genotoxic Diacerein obtained by the process of the invention, in which the total content of genotoxic impurities is below 4ppm, and suitable for the preparation of pharmaceutical formulations for human and veterinary use, in agreement with the current Health Authorities request.

## Description

The present invention concerns a new process for the preparation of Diacetylrhein (Diacerein), with a high degree of purity, specifically with a low content of genotoxic impurities. The invention is also directed to a non-genotoxic Diacerein. These characteristics allow the use of non-genotoxic Diacerein in pharmaceutical formulations for human and veterinary applications.

Diacerein (chemical name: 1,8-diacetoxy-3-carboxyanthraquinone) is a molecule having anti-inflammatory activity (in particular anti-free-radicals activity) and as such can be used in the prevention and treatment of various pathological states, in particular those concerning degradation of cartilage, for example in certain forms of arthritis and osteoarthritis.

Chemically, Diacerein has the following structure:

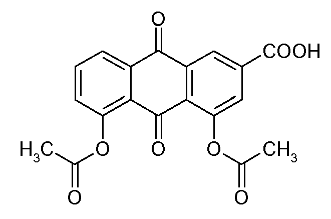

The raw materials for the preparation of Diacerein are vegetable extracts deriving from different species of plants containing the glycosilate-based anthraquinone structure; in particular, Senna or Aloe extracts are the most appropriated. In the following document, the nomenclature used will refer to products obtained from Aloe extracts, although the method could be applied to all vegetable extracts, deriving from any species of plant containing products that can be tranformed into Diacerein.

Usually, the 3-carboxy-1,8-diacetylated anthraquinone product is obtained by an oxidative degradation of the sugars contained in the first-extraction products by different agents such as: chromic anhydride (DE A-4.120.989 and DE A-120.990) or FeCl₃ (WO96/30034). After this oxidation step, the corresponding anthraquinone tri-alcohol (Aloemodine) is isolated; the next step is the acetylation reaction of the phenolic and alcoholic groups present in the Aloemodine with the production of the so-called triacetate (Aloemodine Triacetate). Diacerein is achieved by transforming the methylene-alcoholic group (-CH₂OH) in position 3 into the corresponding carboxylic group by means of oxidation.

An alternative process to obtain crude Diacerin always from Aloin, is the acetylation of Aloin followed by oxidation with chromic anhydride.

As stated above, such steps belong to the state of the art, as described in different patents.

It was already known that crude Diacerein contains impureties, which have to be eliminated.

Many purification processes are described by various Authors. Some patents describe the extraction, with mixtures of organic solvents not particularly mixable (liquid/liquid extraction), sometimes in counter-current (EP 520 414) in the presence of organic bases - such as triethylamine - which increase the solubility of the products. Other Authors carry out successive precipitations of different Inorganic Salts, in various solvent mixtures (WO 2004/050601).

Depending on the process applied, the final product presents a degree of purity that is often unsuitable for pharmaceutical humans and veterinary applications (*per os,* by injection or topic), requiring further purification steps with an obvious reduction in process yield.

In particular, these processes do not lead to a non-genotoxic Diacerein.

In the following, the terms "genotoxic impureties" refer to compounds that have the potential to damage DNA at any level of exposure, said damage leading/contributing to tumour development.

Depending from the vegetables extract used as raw material and applied manufacturing method, different impurities could be present in the final product. Among these impurities, the following products are relevant, due to their genotoxic activity: emodine, monoacetylemodine, diacetylemodine, triacetylemodine, aloemodine, monoacetylaloemodine, diacetylaloemodine and triacetylaloemodine.

When Diacerein is obtained from Aloe, the genotoxic impuritites are Aloemodine, and its mono, di- and tri-acetylated derivatives, which have the following structure:

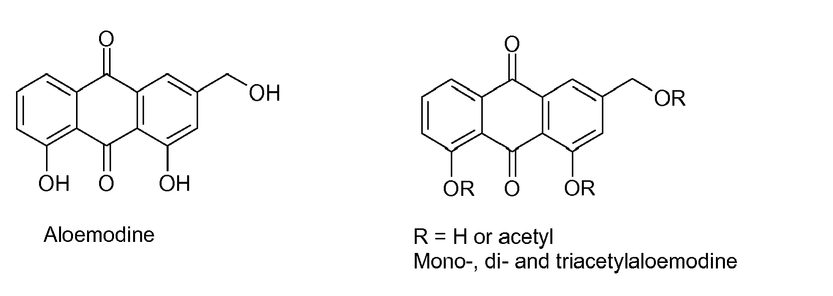

Today, for new drug, Health Authorities ask for identification and a quantification of genotoxic compounds; the maximal accepted amount for genotoxic impurities being fixed by very precise limits.

For example, the ICH guidelines (EMEA/CHMP/QWP/251344/2006), require a total genotoxic impurities content (in term of TTC: Threshold of Toxicological Concern) below to 15 ppm. Specifically, the French Ministry of Health (AFSSAPS) defines for Diacerein, a content of Total Genotoxic Impurity lower than 4 ppm.

Due to the new requirements of Health Agencies on genotoxic compounds, the presence of these impurities make nowadays inappropriate the use of non specifically purified Diacerein as an active ingredient in pharmaceutical preparations for human and veterinary uses. For this reason, it was necessary to develop a new purification process, which will allow obtaining a non genotoxic Diacerein, it means a Diacerein free of these genotoxic impurities.

Surprisingly, it has now been found that appropriate resins can be used to purify Diacerein, with elimination also of the genotoxic compounds (Aloemodine and mono-, di- and triacetylderivatives, in particular Triacetylaloemodine, when Diacerein is obtained from Aloe). This new purification process can be implemented on Diacerein as such or in its de-acetylated form (Rhein). It involves the use of appropriate resins to selectively separate Diacerein, or its derivatives, from the impurities, in particular from genotoxic compounds. This process allows obtaining non-genotoxic Diacerein: preferably genotoxic impurities are not detectable by usual analytical methods (it means less than 1 ppm). The non-genotoxic Diacerein thus obtained can than be used for pharmaceutical formulations for human and veterinary applications. These formulations are in particular appropriate for an administration: *per os, by* injection and topical.

Thus, an object of the invention is a process for producing non-genotoxic Diacerein.

The process of the invention is applied to Diacerein as such or to Rhein. It comprises the following successive steps:
a. transforming raw Diacerein or raw Rhein, into a water-soluble salt thereof; then
b. dissolving said salt in water;
c. adsorbing Diacerein or Rhein on a hydrophobic resin by passage of the aqueous solution obtained in step b. through a column filled with said hydrophobic resin;
d. after step c., desorbing the impurities by flowing an appropriate organic solution through said column and recovering the organic solution which contains the genotoxic impurities;
e. after step d., eluting Diacerein or Rhein by flowing an hydro alcoholic solution through said column and, recovering the hydro alcoholic solution which contains non genotoxic Diacerein or Rhein salt;
f. acidifying the hydro alcoholic solution recovered after step e.;
g. recovering and drying non genotoxic Diacerein.

When the starting product is Rhein, the process comprises, after step f. and before step g., an additional step consisting in transforming Rhein in Diacerein by acetylation.

The terms "non genotoxic Diacerein" means a Diacerein in which the total genotoxic impurities content is below 4 ppm as specifically required from French Ministry of Health (AFSSAPS), preferably below 1 ppm.

The raw Diacerein, used in step a., could have different extraction origins. In particular,
Process of the present invention is applied to:
- the raw material obtained from Aloe which contains Diacerein and impurities (in particular genotoxic impurities), following Type A process described below;
- the mixture obtained by total deacetylation of the above described raw material following Type B Process. De-acetylation to obtain Rhein is performed using known methods, such as those described in WO 96/30034.

The process could start from raw Diacerein or Rhein containing significant amounts of genotoxic impureties.

In a particular embodiment of the invention, when the content of genotoxic impurities is too high (in particular much more than 500 or 600 ppm), at least one previous crystallisation is performed before implementing the process of the invention. The crystallisation solvent can for example be dimethylformamide (DMF).

Surprisingly, the inventors have discovered that lipophylic resins could be used to efficiently separate Diacerein or Rhein from the impurities (particularly the genotoxic impurities) deriving from the extraction/modification of the initial plant extract. Without being bond by any theory, the inventors think that this could be explained by their difference in their degree of lipophylicity/hydrophylicity, correlated to structural differences (for example, presence of acetylated phenolic and primary alcoholic groups or not, or phenolic and acetylated groups or not, and salifiable carboxylic groups).

### Water-soluble salt:

Type A Process: The first step of the process (a.) is to salify the raw Diacerein to obtain a salt of Diacerein which is soluble in water. The water soluble salt of Diacerein is preferably an inorganic salt of Diacerein, more preferably a salt of Diacerein of an alkali metal. In a preferred embodiment, the salt of Diacerein is a potassium salt of Diacerein or a sodium salt of Diacerein. The solubility of potassium salt of Diacerein is around 1g/50ml of water at neutral pH (pH=7).
Type B Process: The first step of the process (a.) is to salify the raw Rhein to obtain a salt of Rhein which is soluble in water. The water soluble salt of Rhein is preferably an inorganic salt of Rhein, more preferably a salt of Rhein of an alkali metal.. In a preferred embodiment, the salt of Rhein is a potassium salt of Rhein or a sodium salt of Rhein.

### Hydrophobic resin:

The resin used in the process of the invention is a hydrophobic resin.

Such hydrophobic resin is preferably a polymer-type, wherein said polymer backbone does not comprise hydrophilic functional groups (such as OH, COOH, NH₂...). The polymer backbone is advantageously an hydrocarbon backbone (constituted from C and H atoms), which may presents hydrophobic-substituents.

A hydrophobic compound does not form hydrogen bounds with the molecules of water. A hydrophobic compound is often a non-polar compound but some hydrophobic compounds can be slightly polar compounds.

As example of suitable polymer, a copolymer of styrene and divinylbenzene (PS-DVB) can be considered. Said copolymer may be substituted on the benzene by halogen atoms (in particular by bromine atom).

According to a preferred embodiment, the resin is not only hydrophobic but is also porous, even more highly porous.

The porosity of the resin may for example be defined by its pore volume, which is preferably above 1 ml/g, more preferably between 1 ml/g and 2.5 ml/g.

The porous hydrophobic resin has advantageously an important specific surface area, which is preferably above 550 m²/g. In a particular embodiment, the specific area is between 550 m²/g and 1300 m²/g, more preferably between 590 m²/g and 1300 m²/g, even more preferably between 590 m²/g and 1200 m²/g.

The resin is preferably under the form of particles, whose size may range from 50 µm to 700 µm In one particular embodiment, the particle size range is between 250 µm and 700 µm, advantageously between 250 µm and 600 µm In another particular embodiment, the particle size ranges between 50 µm and 150 µm.

The resin may furthermore have a tendency to swell. In a particular embodiment, a resin having a water retention capacity ranging from 40% to 70% is used, advantageously from 45% to 55%.

As non limiting examples of appropriate commercial resins, the following products could be mentioned: DIAION® (HP20, HP20SS, HP21, HP2MGL) or SEPABEADS® (SP70, SP700, SP85, SP850, SP20SS, SP207, SP207SS) or MCI® GEL (CHP20A/Y/P, CHP55A/Y) or other PS-DVB resins.

Since the purification process is based on interactions between the compounds (Diacerein or Rhein and impurities) and the resin, a sufficient contact time between them is essential. The person skilled in the art, on the basis of Producer instructions and preliminary tests, is able to define the column dimensions (length, diameter), the ratio between the amount of the product to be purified and the resin, and other parameters (solvent, flow, pressure...) to perform the purification process. The person skilled in the art will also take into consideration the quantity of impurities contained in the raw Diacerein or Rhein.

### Separation process:

The water soluble salt of raw Diacerein (or Rhein) is dissolved in water (step b.) and then the resulting aqueous solution is percolated through a column filled with a hydrophobic resin (step c.) prepared according to the instructions described by the Producer and preliminary experiment results.

In the following, the column filled with a hydrophobic resin is also called a "resin bed". The resin can also be designated by the use of the term "adsorbent".

In a preferred embodiment, the purification process starts with the percolation of the Diacerein (or Rhein) sodium salt aqueous solution, through the resin bed, at the appropriate speed, chosen by preliminary studies. The percolation of the aqueous solution, through the resin bed, results on interactions between the molecules (Diacerein -or Rhein- and impurities) and the adsorbent; the interaction forces with the adsorbent being different for Diacerein (or Rhein) or impurities. This first step is called "adsorption step".

During the adsorption step, Diacerein (or Rhein) and impurities are adsorbed on the resin. The aqueous solution from the column does not contain significant amounts of Diacerein (or Rhein) and impurities any more.

After the adsorption step, the resin bed is washed with an appropriate organic solvent, in particular polar organic solvent such as acetone or acetonitrile. This second step is called "washing step" (step d.). This washing step will allow desorption of the impurities with the consequence that the organic solution from the column contains all the impurities but does not contain significant amounts of Diacerein (or Rhein). After the washing step, the only compounds still adsorbed on the resin are Diacerein or Rhein.

The following step is called "elution step" (step e.): the elution solvent (also called eluant) is pecolated through the column, one or many times (at least two times). This elution step will allow the recovery of Diacerein or Rhein from the column.

In a preferred embodiment, the eluant is a mixture of water and alcohol, the alcohol being preferably a C₁-C₄ alcohol, more preferably methanol or ethanol. The alcohol/water ratio preferably ranges from 10%/90% to 60%/40% (the percentages being expressed in weight compared to the total weight of water and alcohol). In a preferred embodiment of the invention, at least two elution steps are performed with hydro alcoholic solutions, using a gradient of water/alcohol, starting from the lower percentage of alcohol. In a more preferred embodiment of the invention, the first elution is performed by an ethanol or methanol/water mixture in about 20/80 ratio and the last elution is performed by an ethanol or methanol/water mixture in about 60/40 ratio.

The number of elution steps, which depends on the nature of the resin and of the eluant, can easily be determined by the person skilled in the art with preliminary tests on selected resin.

The first fraction of eluates which contain the Diacerein or the Rhein salts is recovered and if appropriate, also the following fractions, containing additional Diacerein or Rhein. The detection and the quantification of Diacerein (or Rhein) in the eluted fractions are determinated by HPLC (254nm) using reference standards and related retention times.

When Diacerein or Rhein from Aloe is purified by this process, the HPLC method can have for example the following main characteristics:
Column: Supelcosil LC-ABZ®150x4.6 mm, 5 µm (Supelco)
Flow rate: 1.5 mL/min
Detector: 254 nm
Column temperature: 40°C
Injection volume: 20 µl
Time: 25 min
LOD: 0.45 ppm (w/w) with regards to Diacerein
LOQ: 1.35 ppm (w/w) with regards to Diacerein

More precise data can be obtained by HPLC coupled with a mass spectrometry detector.

The recovered fractions, containing Diacerein (or Rhein) salts, are then acidified by well-known methods in order to obtain the Diacerein (or Rhein) as free carboxylic acid (step f.). Rhein is transformed into Diacerein by described methods. Afterward, the non-genotoxic Diacerein is recovered and dried by usual method (step g.).

Diacerein in form of the free carboxylic acid can be recovered by acidification with an appropriate organic or inorganic acid (preferably a diluted strong acid such as H₂SO₄ 1M), with subsequent precipitation of the Diacerein, its filtration under vacuum, followed by washings with water. In the case of Rhein, after acidification with an appropriate organic or inorganic acid (preferably a diluted strong acid such as H₂SO₄ 1M), the precipitate is filtered and, after complete drying, is dissolved in anhydrous pyridine and acetic anhydride (as described by well-know methods). Once reaction is completed, the addition of water and ice to the mixture causes the precipitation of non-genotoxic Diacerein and the dissolution of the present salts. The final product is recovered by filtration, preferably under vacuum or centrifugation, followed by washings with water.

The degree of purity of the final product is detected by HPLC following for example the chromatographic conditions described above.

The genotoxic impurities content is advantageously below the detection threshold of this method, which means that the content is below than 1 ppm.

The total yield of Processes A or B is on average around 85-95% referring to the raw Diacerein or raw Rhein content, in form of the free carboxylic acid, which may contain even more than 500-600 ppm of genotoxic impurities.

The invention is also directed to non-genotoxic Diacerein obtainable by the process of the invention. In said non-genotoxic Diacerein the total content of genotoxic impurities is below 4 ppm, advantageously below 1 ppm.

The invention also encompasses pharmaceutical formulations comprising as active ingredient said non-genotoxic Diacerein. The formulations are preferably under a form suitable for an administration by oral, injection or topical route.

The non-genotoxic Diacerein of the invention can be used as medicament, for human beings and animals.

The following examples, which in no way limit the scope of the invention, illustrate the preferred embodiments of the processes.

### EXAMPLE 1 (Use of SEPABEADS SP207® and HP20® resins)

### Preparation of Potassium salt of raw Diacerein

50g of raw Diacerein containing 500 ppm of genotoxic impurities are suspended in 750ml of acetone and 50ml of water and, under magnetic agitation, 25ml of triethylamine diluted in 100ml of acetone, are added over a period of 3 hours, keeping pH not more than 7, until complete dissolution. The final solution obtained is treated at 18°C with 32g of Potassium Ethyl Hexanoate in 260 ml of acetone. Salification agent is added over a period of 2 hours. A precipitate is formed; after filtration, the precipitate is washed with 500ml of acetone and dried under vacuum at 40°C for one night.

50g of Potassium Salt of Diacerein are obtained.

### Elution:

15g of this product are dissolved in 750ml of water. This solution, after filtration under vacuum, is percolated through a 4.5cm-diameter 120cm-high column, packed with 1.1L of SEPABEADS® SP207® or DIAION HP20® (flow rate 20ml/min).

The typical characteristics of SEPABEADS® SP207® and DIAION HP20® are given in the table 1 below:

**Table 1: characteristics of SEPABEADS® SP207® and DIAION HP20®**

| | SEPABEADS® SP207® | DIAION HP20® |
|---|---|---|
| Water retention % | 45 -55 | 55-65 |
| Particle size 205 µm | 250 - 600 | 250-600 |
| Specific surface area (m²/g) | 590 | 590 |
| Specific gravity | 1.18 | 1.01 |
| Pore volume (ml/g) | 1.1 | 1.3 |
| Average pore radius (Å) | 120 | 260 |

Afterwards, the column is washed with a bed volume of acetone (flow rate 18-20 ml/min).

Four elution steps with ethanol/water mixture are performed to recover Diacerein Potassium salt: the first elution is performed by using ethanol/water mixture 20%/80% (100ml/400ml) and the three last elutions by using ethanol/water mixture 60%/40% (300ml/200ml) (for each elution step the flow rate is about 15-20 ml/min).

The collected fractions, containing the product, are then brought to pH 2.5-3 with 10% sulphuric acid (H₂SO₄). The suspension is cooled to 20°-25°C and stirred for a period of 30 minutes; the precipitate is recovered by filtration under vacuum, washed with 150ml of hot water (45-50°C) and 150ml of acetone, and then dried under vacuum.

Around 11g of Diacerein are obtained with both the cited resins, showing a genotoxic impurities content lower than 1 ppm detected by HPLC (0.95ppm or 0.50ppm of genotoxic impurities content respectively).

### EXAMPLE 2 (Use of DIAION HP2MG resin)

### Preparation of Potassium salt of raw Diacerein

50g of Diacerein containing 500ppm of genotoxic impurities are suspended in 750ml of acetone and 50ml of water and, under magnetic agitation, 25ml of triethylamine diluted with 100ml of acetone are added over a period of 3 hours keeping pH not more than 7, until complete dissolution. The obtained solution is treated with 32g of Potassium Ethyl Hexanoate in 260ml of acetone, over a period of 2 hours. The obtained precipitate is filtered, washed with 500ml of acetone and dried under vacuum at 40°C for one night.

50g of Potassium Diacerein salt is obtained.

### Elution:

25g of Potassium Diacerein salt are dissolved in 1250ml of water. The solution, after filtration under vacuum, is percolated through a 10.0 cm-diameter and 110 cm high column, packed with 5.1 L of DIAION HP2MG® (flow rate 20 ml/min).

The typical characteristics of DIAION HP2MG®are given in the table 2 below:

**Table 2: characteristics of DIAION HP2MG®.**

| | DIAION HP2MG® |
|---|---|
| Water retention % | 55-65 |
| Particle size 205 µm | 300-700 |
| Specific surface area (m²/g) | 570 |
| Specific gravity | 1.09 |
| Pore volume (ml/g) | 1.3 |
| Average pore radius (Å) | 240 |

The column is washed with a bed volume of acetone (flow rate 18-20 ml/min), followed by 4 elutions steps: the first elution step using ethanol/water mixture 20%/80% (100ml /400ml) and the last three steps with ethanol/water 60%/40% (300ml/200ml) (for each elution step the flow rate is about 15-20 ml/min).
The collected fractions containing the product are then brought to pH 2.5-3.0 with sulphuric acid. The suspension is cooled to 20°-25°C and stirring over a period of 30 minutes. The precipitate is filtrated under vacuum, washed with 300ml of hot water (45°-50°C) and 150ml of acetone, and then dried under vacuum.

6.25 g of Diacerein are obtained, showing a genotoxic impurity content lower than 1 ppm (0.60 ppm of genotoxic impurities).

### EXAMPLE 3

5g of raw Diacerein containing about 300 ppm of genotoxic impurities derivatives is dissolved in 40ml of methanol and, under magnetic agitation, 40ml of water and 5g of KOH are added. In the presence of a condenser, heating to 60-65°C is performed for 30 minutes; after this period, 35ml of 6N HCL are added; dilution with about 35ml of water is performed and the solution is boiled for about 30 minutes. After cooling, the suspension is filtered under vacuum, the residue washed with water and dried under vacuum at constant weight.

4.5g of Rhein are thus obtained.

2g of Rhein thus obtained are transformed into the corresponding potassium salt as described for the Diacerein in Example 1.

2g of Potassium Salt of Rhein are dissolved in 200ml of water (final pH of the solution 6.2). This solution, after filtration under vacuum, is percolated through a 7.5cm-diameter 10cm-high column, packed with 180g of SEPABEADS® SP207® (flow rate 20 ml/min).

Washing with a volume corresponding to the volume of the column of acetone (flow rate 18-20 ml/min) and then elution with a water/ethanol mixture are performed until the complete elution of the Rhein. 4 elution steps are performed: the two first elutions are performed by using ethanol/water mixture 20%/80% and the two last elutions are performed by using ethanol/water mixture 60%/40% (for each elution step the flow rate is about 15-20 ml/min).

The fraction containing the Rhein is then brought to pH 4.5-5 with 10% sulphuric acid (H₂SO₄). The suspension is cooled to 5-10°C, the precipitate recovered by filtration under vacuum, washed with cold water and dried under vacuum.

The precipitate, after drying, is acetylated using pyridine and acetic anhydride in a ratio of 1:1 (alternatively, other conventional acetylating agents may be used).

After drying, 1.5g of Diacerein are obtained, showing a content of genotoxic impurities lower than 1ppm (0.80ppm of genotoxic impurities).

## Claims

1. A process for producing non-genotoxic Diacerein, starting from raw Diacerein or raw Rhein, comprising the following successive steps:
a. transforming raw Diacerein or raw Rhein into a water-soluble salt thereof; then
b. dissolving said salt in water;
c. adsorbing Diacerein or Rhein on a hydrophobic resin, by percolation of the aqueous solution obtained after step b. through a column filled with hydrophobic resin;
d. after step c., desorbing the impurities using an organic solution, recovering the organic fractions which contain the impurities;
e. after step d., desorbing Diacerein or Rhein using an hydro alcoholic solution, recovering the hydro alcoholic fractions which contain Diacerein or Rhein salt;
f. acidifying the collected hydro alcoholic fractions recovered after step e.;
g. recovering and drying non-genotoxic Diacerein.

2. The process according to claim 1, further comprising, after step f. and before step g., an additional step consisting in transforming Rhein in Diacerein by acetylation.

3. The process according to claims 1 or 2, wherein in step a. the water soluble salt is an inorganic salt, preferably a salt of an alkali metal.

4. The process according to claim 3, wherein in step a. the water soluble salt is a potassium salt or a sodium salt.

5. The process according to anyone of claims 1 to 4, wherein in step c. the hydrophobic resin is a copolymer of styrene and divinylbenzene (PS-DVB), which may be substituted on the benzene by halogen atoms.

6. The process according to anyone of claims 1 to 5, wherein in step c. the hydrophobic resin is porous; the pore volume being preferably above 1ml/g, more preferably between 1ml/g and 2.5ml/g.

7. The process according to anyone of claims 1 to 6, wherein in step c. the hydrophobic resin has a specific surface area, which is above 550 m²/g, preferably between 550 m²/g and 1300 m²/g, more preferably between 590 m²/g and 1300 m²/g, even more preferably between 590 m²/g and 1200 m²/g.

8. The process according to anyone of claims 1 to 7, wherein in step c. the hydrophobic resin is under the form of particles, and the particle size range is from 50 µm to 700 µm, preferably between 250 µm and 700 µm, more preferably between 250 µm and 600 µm.

9. The process according to anyone of claims 1 to 8, wherein in step c. the hydrophobic resin has a water retention capacity ranging from 40% to 70%, advantageously from 45% to 55%.

10. The process according to anyone of claims 1 to 9, wherein step e. is repeated at least two times and each hydro alcoholic fraction is collected.

11. The process according to anyone of claims 1 to 10, wherein step e. is performed with the use of water/ethanol or water/methanol mixtures as elution solvent.

12. The process according to anyone of claims 1 to 11, wherein in step e. the alcohol/water mass ratio ranges from 10%/90% to 60%/40% (w/w).

13. Non-genotoxic Diacerein obtainable by the process according to anyone of claims 1 to 12, in which the total content of genotoxic impurities is below 4 ppm.

14. Pharmaceutical formulations comprising as active ingredient the non-genotoxic Diacerein of claim 13, said formulations being preferably under a form suitable by an administration by oral, injection or topical route.

15. Non-genotoxic Diacerein of claim 13 for its use as medicament, for human beings and animals.
